# EUROPEAN PATENT APPLICATION

(11) **EP 2 546 334 A1**
(43) Date of publication of application: **16.01.2013**
(21) Application number: 11752901.6
(22) Date of filing: 10.03.2011
(51) Int. Cl.: C12N 5/071

(54) **METHOD FOR THE IN VITRO PROLIFERATION OF CELLS DERIVED FROM TISSUES OF ENDORMAL ORIGIN**

(30) Priority: 12.03.2010 ES 201030365
(71) Applicant: Fundación Progreso Y Salud, 41092 Sevilla (ES); Universidad Pablo de Olavide, 41013 Sevilla (ES)
(72) Inventor: KHOO, Adrián, Parque Científico y Tecnológico Cartuia 93 41092 Sevilla (ES); SORIA ESCOMS, Bernat, Parque Científico y Tecnológico Cartuia 93 41092 Sevilla (ES); MARTÍN BERMUDO, Franz, 41013 Sevilla (ES)
(74) Representative: Illescas, Manuel
(86) International application number: PCT/ES2011/070165
(87) International publication number: WO 2011/110722

(57) **Abstract**

The present invention relates to a method for the rapid *in vitro* proliferation of cells derived from tissues of endodermal origin, preferably pancreatic beta cells. It also relates to the proliferation-inducing cell culture medium used in said method, to the cells and cell populations obtainable thereby, and to the drugs that comprise these cells or cell populations for use in somatic cell therapy for injuries or diseases of endoderm-derived tissues, preferably injuries or diseases of the pancreas; more preferably, diabetes mellitus.

## Description

The present invention falls within the field of biomedicine. Specifically, it relates to a method for the rapid *in vitro* proliferation of cells derived from tissues of endodermal origin, preferably pancreatic beta cells. It also relates to the proliferation-inducing cell culture medium used in said method, to the cells and cell populations obtainable thereby, and to the drugs that comprise these cells or cell populations for use in somatic cell therapy for injuries or diseases of endoderm-derived tissues, preferably injuries or diseases of the pancreas, more preferably diabetes mellitus.

### PRIOR STATE OF THE ART

Edmonton's protocol (Shapiro AMJ., et al., 2000, The New England Journal of Medicine, 343: 230-238) for the transplantation of pancreatic islets from cadaver donours has proven to be an effective alternative for the treatment of diabetes mellitus, by means of cell therapy. Unfortunately, the number of pancreatic islets that are isolated from cadaver donours is low and this entails the need to have pancreata from 2-3 cadaver donours available in order to guarantee the success of the islet transplantation. Although the number of donours has increased in recent years, it is clearly insufficient to guarantee islet transplantation for a significant number of diabetes patients. A potential alternative to overcome this problem would be the development of protocols that allow for the *in vitro* proliferation of pancreatic beta cells prior to the transplantation. This would increase the mass of beta cells to be transplanted.

The protocols developed thus far to increase the proliferation of beta cells are primarily based on modifying the *in vitro* culture conditions. In this regard, the growth factors or the extracellular matrices added to the culture media are modified (Beattie, GM., et al., 1997, Journal of Clinical Endocrinology & Metabolism, 82: 1852-1856). However, these processes have not made it possible to increase the proliferation of beta cells by more than 2% every 24 h. Moreover, many of these protocols lead to an irreparable loss of the phenotype and the function of beta cells (Ouziel-Yahalom L., et al., 2006, Biochemical and Biophysical Research Communications, 341: 291-298), which prevents the subsequent clinical application thereof.

On the other hand, the disdifferentiation of epithelial cells has been associated with a phenomenon called epithelial-mesenchymal transition (EMT). Under certain culture conditions, pancreatic beta cells may undergo EMT, disdifferentiate towards fibroblast-like cells, proliferate and finally re-differentiate towards cells that express insulin and glucagon (Ouziel-Yahalom L., et al., 2006, Biochemical and Biophysical Research Communications, 341: 291-298; Gershengorn MC., et al., 2004, Science, 306: 2261-2264). The EMT does not induce multipotent properties in disdifferentiated pancreatic beta cells, which indicates that these cells retain a restricted differentiation potential and, therefore, are more susceptible to re-differentiate towards functional beta cells (Russ HA., et al., 2009, Plos One, 4: e6417).

It has been demonstrated that the signals sent by the blood vessels play a significant role in the proliferation and specification of the identity of hepatic and pancreatic cells (Lammert E., et al., 2001, Science, 294: 564-567). This is partly due to the secretion of growth factors and cytokines by vascular endothelial cells (Johansson M., et al., 2006, Endocrinology, 147: 2315-2324), as well as the extracellular matrix secreted by the aforementioned cells (Nikolova G., et al., 2006, Developmental Cell, 10: 397-405).

In summary, although efforts have been undertaken to improve the *in vitro* culture conditions of pancreatic beta cells, in order to achieve an increase in cell density and thus have available a sufficient number of cells for transplantation, within treatment methods based on cell therapy, there is still a need to develop *in vitro* culture methods that improve the proliferation of these cells. This will make it possible to obtain a greater cell mass in a shorter period of time, as compared to conventional methods, and, moreover, will prevent the loss of the phenotype and the biological functionality of the cells during their expansion under culture.

### DESCRIPTION OF THE INVENTION

The present invention provides a method for the rapid *in vitro* proliferation of cells derived from tissues of endodermal origin, preferably pancreatic beta cells. It also relates to the proliferation-inducing cell culture medium used in said method, to the cells and cell populations obtainable thereby, and to the drugs that comprise these cells or cell populations for use in somatic cell therapy for injuries or diseases of endoderm-derived tissues, preferably injuries or diseases of the pancreas, more preferably diabetes mellitus.

The method developed in the invention involves establishing a culture rich in cells derived from tissues of endodermal origin, preferably pancreatic beta cells or hepatocytes, by culturing them in the presence of a proliferation-inducing cell culture medium, and the subsequent co-culture thereof with inactivated endothelial cells. This method allows for a rapid proliferation of endodermal cells *in vitro,* preventing the complete disdifferentiation thereof and, consequently, their loss of functionality. As shown in the examples of the present invention, the cells obtainable by means of this *in vitro* cell proliferation method present an endocrine precursor cell phenotype. The proliferation rate of adult murine pancreatic beta cells is 0.5%-1 % and, when they are forced to proliferate, the increase achieved is not greater than 10% and takes place at the expense of the loss of their beta-cell phenotype. Using the *in vitro* cell proliferation method of the invention, it has been possible to go from a culture under control conditions with 69% ± 3% of beta cells to a culture with 98% ± 2% beta cells in approximately 14 days. Similarly, the increase in cell proliferation observed for beta cells is 4.02 ± 0.3 times, and 2.8 ± 0.2 times for hepatocytes. This means that the *in vitro* cell proliferation method of the invention makes it possible to expand the mass of pancreatic beta cells, and, in general, of cells derived from tissues of endodermal origin, more than the existing protocols and, moreover, they preserve their phenotype, which is especially relevant in cell therapy for diseases such as diabetes, where the main problem is the lack of pancreatic beta cells for transplantation.

Therefore, one aspect of the invention relates to a DMEM cell culture medium, hereinafter "culture medium of the invention" or "proliferation-inducing culture medium", supplemented with:
a. between 4% and 10% of "foetal calf serum" or "CF",
b. between 90 and 110 U/ml of penicillin, and between 80 and 120 pg/ml of streptomycin,
c. between 0.5 and 2 mM of sodium pyruvate,
d. between 0.05 and 0.2 mM of 2-beta-mercaptoethanol,
e. between 0.05 and 0.2 µg/ml of hydrocortisone,
f. between 4 µg-4 µg-4 ng/ml and 6 µg-6 µg-6 ng/ml of insulin-transferrin-selenium,
g. between 900 and 1,100 U/ml of leukemia inhibitory factor or "LIF",
h. between 1 and 2 mM of EGTA,
i. between 0.5% and 2% of non-essential amino acids, and
j. between 9 and 11 ng/ml of epidermal growth factor or "EGF".

"DMEM" medium, or "Dulbecco's modified Eagle's medium", is a cell culture medium designed for the growth of mammalian cells, the composition whereof is known to persons skilled in the art. In a preferred embodiment, this DMEM medium comprises between 0.5 g/I and 1.5 g/I of glucose. In a more preferred embodiment of this aspect of the invention, the DMEM medium comprises 1 g/I of glucose, a concentration which, as shown by the examples of the present invention, has proven to be optimal for the culture of pancreatic beta cells.

For the culture of other cell types derived from tissues of endodermal origin, such as, for example, without being limited thereto, hepatocytes, the DMEM concentration which, as shown by the examples of the present invention, has proven to be optimal for the culture of cells is 4.5 g/I of glucose. Therefore, in another preferred embodiment, the DMEM medium comprises 4.5 g/l of glucose when it is to be used, for example, without being limited thereto, for the culture of hepatocytes. In a more preferred embodiment, for said culture of hepatocytes, DMEM 4.5 g/I of glucose with glutamax (between 2 and 4 mM), sodium pyruvate (between 0.1 and 1 mM) and HEPES (between 10 and 20 mM) is used, supplemented with elements (a) to (h) of the culture medium of the invention. "Glutamax" is a cell culture medium with a composition known to persons skilled in the art and formed by L-alanyl-L-glutamine. "Pyruvate" is the carboxylate anion of pyruvic acid. "HEPES" is N-2-hydroxyethylpiperazine-N'-2'-ethanesulfonic acid.

EGTA is the chemical compound with number CAS 67-42-5. The non-essential amino acids that may be used in the culture medium of the invention are, preferably, glycine, L-alanine, L-asparagine, L-aspartic acid, L-glutamic acid, L-proline and L-serine.

In another preferred embodiment, the culture medium of the invention comprises DMEM 1 g/I glucose supplemented with: 5% of foetal calf serum, 100 U/ml-100 µg/ml of penicillin-streptomycin, 1 mM of sodium pyruvate, 1% of non-essential amino acids, 0.1 mM of 2-beta-mercaptoethanol, 0.1 µg/ml of hydrocortisone, 5 µg-5 µg-5 ng/ml of insulin-transferrin-selenium, 1,000 U/ml of leukemia inhibitory factor, 10 ng/ml of epidermal growth factor and 1.5 mM of EGTA; these quantities, as shown by the examples of the present invention, have proven to be optimal for the culture of pancreatic beta cells.

In another preferred embodiment, the culture medium of the invention comprises DMEM 4.5 g/I glucose, glutamax (2 mM), sodium pyruvate (1 mM) and HEPES (10 mM), supplemented with: 10% of foetal calf serum, 100 U/ml-100 pg/ml of penicillin-streptomycin, 0.1 mM of 2-beta-mercaptoethanol, 0.1 µg/ml of hydrocortisone, 5 µg-5 µg-5 ng/ml of insulin-transferrin-selenium, 1,000 U/ml of leukemia inhibitory factor and 1.5 mM of EGTA; these quantities, as shown by the examples of the present invention, have proven to be optimal for the culture of hepatocytes.

The optimal quantities of each of the elements wherewith the culture medium of the invention is supplemented may vary within experimentally reasonable ranges depending on the cell type to be cultured; for this reason, the quantities of such elements, as well as the physical-chemical parameters of the culture, will be experimentally adjusted as a function of the cell type derived from a tissue of endodermal origin to be cultured.

Another aspect of the invention relates to a method for obtaining cells from isolated tissues of endodermal origin, hereinafter "method of the invention", which comprises:
a. isolating the cells of a biological sample isolated from a tissue of endodermal origin of a mammal by means of a collagenase digestion protocol,
b. culturing the cells isolated in step (a) in the presence of the culture medium of the invention for 70-74 hours,
c. adding inactivated endothelial cells to the culture of step (b), and
d. co-culturing the culture of step (b) and the inactivated endothelial cells added in step (c) for 10-12 days.

In a preferred embodiment of this aspect of the invention, the density of the cells cultured in step (b) is between 90,000 and 110,000 cells/cm² at the start of the culture. In a more preferred embodiment, the density of the cells cultured in step (b) is 100,000 cells/cm² at the start of the culture. In another preferred embodiment, the density of the inactivated endothelial cells of step (c) is between 50,000 and 70,000 cells/cm² at the time of addition to the culture of step (b). In a more preferred embodiment, the density of the inactivated endothelial cells of step (c) is 60,000 cells/cm² at the time of addition to the culture of step (b).

In another preferred embodiment, the tissue of endodermal origin of step (a) is pancreatic tissue. In a more preferred embodiment, the isolated biological sample of step (a) is an islet of Langerhans. In an even more preferred embodiment, the cells isolated in step (a) are pancreatic beta cells. In another preferred embodiment, the inactivated endothelial cells of step (c) are derived from the Mil Seven 1 cell line.

In another preferred embodiment, the tissue of endodermal origin of step (a) is hepatic tissue. In a more preferred embodiment, the cells isolated in step (a) are hepatocytes. In an even more preferred embodiment, the inactivated endothelial cells of step (c) are derived from the liver.

In the present invention, "tissues of endodermal origin" are understood to mean those tissues that are a part of the organs selected from the list that comprises: pancreas, trachea, bronchi, lungs, liver, bladder, digestive system, thyroid, thymus, tympanic cavity, auditory tube, tonsils or parathyroid. A "cell derived from a tissue of endodermal origin" is any cell comprised in said endodermal tissues.

An isolated biological sample includes, without being limited thereto, tissues and/or biological fluids derived from a tissue of endodermal origin of a mammal, obtained by any method known to persons skilled in the art which serves that purpose. Moreover, the isolated biological sample may be, for example, without being limited thereto, fresh or frozen. Preferably, the biological sample isolated from a tissue of endodermal origin of a mammal is derived from a human being, more preferably a human cadaver.

The isolation of the cells of the isolated biological sample of step (a) is performed by means of a collagenase digestion protocol. Collagenase digestion protocols for the isolation of cells are known in the state of the art. Thus, for example, without being limited thereto, in the event that the tissue of endodermal origin of step (a) of the method of the invention is hepatic tissue, the hepatocyte isolation protocol is Seglen's protocol (Seglen, P.O., 1976, Methods in Cell Biology, vol. XIII, David M. Prescott ed., Academic Press, 4: 29-83). In the event that the tissue of endodermal origin of step (a) of the method of the invention is pancreatic tissue and the isolated biological sample derived therefrom are islets of Langerhans, said collagenase digestion protocol for the isolation of pancreatic islets is, preferably, the protocol developed by Lernmark (Lernmark A., 1974, Diabetologia, 10: 431-438), with the modifications explained below. Thus, in another preferred embodiment, the collagenase digestion protocol of step (a) of the method of the invention, hereinafter "digestion protocol of the invention", comprises:
a. preparing a solution that comprises 115 mM NaCl, 5 mM KCl, 10 mM NAHCO₃, 1.1 mM MgCl₂, 1.2 mM NaH₂PO₄, 1 mM CaCl₂, between 0.5 and 1 mM of glucose, and between 0.5% and 3% of bovine albumin, at 4°C and pH 7.3,
b. adding between 5 and 8 mg of type V collagenase to between 6 and 10 ml of the solution of step (a),
c. placing the pancreas, previously extracted from a mammal that has been injected with between 2.5 and 5 ml of the solution of step (b), in a container with between 5 and 9 ml of collagenase,
d. incubating the container of step (c) at 37°C and stirring it for 15 seconds at minutes 5, 7 and 9,
e. stopping the incubation of step (d) at minute 10 by the addition of between 3 and 6 ml of the solution of step (a),
f. centrifuging the solution obtained in step (e) twice at 200 g for between 3 and 5 minutes at room temperature and eliminating the supernatant in each centrifugation,
g. isolating the pancreatic islets present in the solution obtained in step (f), by manual picking with a pipette under a stereoscopic magnifying glass, and placing them in a container with the solution of step (a),
h. adding a phosphate buffer solution that comprises between 0.5% and 3% of bovine albumin, between 1 and 3 mM glucose, and 1.1 mM MgCl₂ to the container of step (g),
i. centrifuging the solution of step (h) at 50 g for between 1 and 3 minutes at room temperature, and eliminating the supernatant,
j. adding between 2 and 4 ml of a non-enzymatic cell dissociation solution to the solution obtained in step (i) at 37°C,
k. incubating the solution of step (j) at 37°C for 8 minutes and mechanically dispersing the pancreatic islets in the solution by means of 10 pipettings at minutes 2, 4 and 6 of said incubation,
l. adding between 0.5 and 1.5 ml of a trypsin-EDTA solution to the solution of step (k) during the last minute of the incubation,
m. adding between 3 and 6 ml of DMEM culture medium to the solution obtained in step (I),
n. centrifuging the solution of step (m) at 200 g for between 3 and 5 minutes at room temperature, and eliminating the supernatant,
o. adding between 1 and 2 ml of the culture medium of step (m) to the solution obtained in step (n),
p. filtering the solution obtained in step (o) through a 70-µm mesh,
q. centrifuging the filtrate obtained in step (p) at 200 g for between 3 and 5 minutes at room temperature, and
r. seeding the cells obtained following the centrifugation of step (q).

In step (a) of the digestion protocol of the invention, an islet isolation solution is prepared, which must be, preferably, at 4°C. The composition of the buffer of said solution is preferably (in mM): 115 NaCl, 5 KCl, 10 NAHCO₃, 1.1 MgCl₂, 1.2 NaH₂PO₄, 1 CaCl₂ and between 0.5 and 1 glucose, preferably 0.5 mM of glucose. Between 0.5% and 3% of bovine albumin is added to this solution, preferably 0.5% of, for example, without being limited thereto, Sigma bovine albumin, and the pH thereof is adjusted to 7.3. Between 6 and 10 ml, preferably 8 ml, of this solution are collected, and between 5 and 8 mg, preferably 6.3 mg, of type V collagenase are added thereto, for example, without being limited thereto, from Sigma (collagenase activity 533 units/mg). Between 2.5 and 5 ml, preferably 3 ml, of the cold collagenase solution (solution of step (b) of the digestion protocol of the invention) are injected in the bile duct of a mammal and, after the pancreas is swollen, it is extracted as quickly as possible, removing the fatty tissue and the connective tissue. Subsequently, in step (c) of the digestion protocol of the invention, the pancreas is placed in a container, preferably a tube, with between 5 and 9 ml, preferably 7 ml, of collagenase. Subsequently, in step (d) of this protocol, the tube with the pancreas is introduced into an incubation bath at 37°C. The pancreas is vigorously stirred by hand, for 15 seconds, at minutes 5, 7 and 9. The incubation is stopped, in step (e) of this protocol, at minute 10 by the addition of between 3 and 6 ml, preferably 5 ml, of isolation solution at 4°C, without collagenase (solution of step (a) of the digestion protocol of the invention). The resulting solution is centrifuged 2 times at 200 g for between 3 and 5 minutes, preferably for 5 minutes, at room temperature, in step (f) of this protocol. In each centrifugation, the supernatant is eliminated. Finally, in step (g) of the digestion protocol of the invention, the pancreatic islets present in the solution obtained in step (f) are isolated by manual picking using a pipette, under a
stereoscopic dissecting magnifying glass, and placed in a container, preferably a Petri dish, with cold isolation solution without collagenase (solution of step (a) of the digestion protocol of the invention). The pancreatic islets that are not completely separated from the exocrine tissue or the vascular tissue are manually dissected, preferably with two insulin syringes. Following the isolation of the pancreatic islets, these are washed, in step (h) of the digestion protocol of the invention, by adding a phosphate buffer solution, preferably a Dulbbeco phosphate buffer solution, comprising between 0.5% and 3%, preferably 1%, of bovine albumin, between 1 and 3 mM, preferably 2.5 mM, of glucose and 1.1 mM MgCl₂, to the container of step (g) that comprises the islets. The islets in this wash solution are centrifuged once at 50 g, for between 1 and 3 minutes, preferably for 1 minute, at room temperature, in step (i) of the digestion protocol of the invention, and the supernatant is eliminated. Subsequently, in step (j) of this protocol, between 2 and 4 ml, preferably 3 ml, of a non-enzymatic cell dissociation solution are added at 37°C, for example, without being limited thereto, the Sigma non-enzymatic cell dissociation solution, catalogue no. C5914. Subsequently, the solution obtained in step (j) is incubated for 8 minutes in an incubation bath at 37°C, in step (k) of the digestion protocol of the invention. During the incubation time, the islets are mechanically dispersed by pipetting 10 consecutive times, preferably with a 1-ml pipette, at minutes 2, 4 and 6 of said incubation. The pipetting must be soft, taking care not to produce bubbles. During the last minute of the incubation, between 0.5 and 1.5 ml, preferably 1 ml, of a trypsin-EDTA solution are added, such as, for example, without being limited thereto, the trypsin-EDTA (Gibco) 0.1 x solution, and the mechanical dispersion is continued by means of pipetting, in step (I) of the digestion protocol of the invention. In order to stop the dissociation process of the pancreatic islets into cells, in step (m) of this protocol between 3 and 6 ml, preferably 5 ml, of DMEM culture medium are added, for example, without being limited thereto, DMEM (Gibco), containing 4.5 g/I of glucose and supplemented with 10% of foetal bovine serum, for example, without being limited thereto, foetal bovine serum (Hyclone). Subsequently, in step (n) of this protocol, the cell suspension comprised in the solution of step (m) is centrifuged at 200 g for between 3 and 5 minutes, preferably for 5 minutes, at room temperature, the supernatant is eliminated and, in step (o), between 1 and 2 ml, preferably 1 ml, of the aforementioned culture medium, also added in step (m), are added. Subsequently, in step (p), the cell suspension is filtered through a 70-µm mesh, for example, without being limited thereto, a Becton Dickinson mesh, preferably using a 1-ml pipette at high velocity. Following the filtration, the cell suspension is once again centrifuged, in step (q), at 200 g for between 3 and 5 minutes, preferably for 5 minutes, at room temperature, and, finally, the cells are seeded in step (r) of the digestion protocol of the invention.

The times and the quantities of the products comprised in the digestion protocol of the invention may vary within experimentally reasonable ranges, due to the potential variations inherent in the implementation of said method.

In order to perform the culture of step (b) of the method of the invention, the cells isolated in step (a) are seeded, preferably at a cell density ranging between 90,000 and 110,000 cells/cm², and, more preferably, at a cell density of 100,000 cells/cm², in a culture container, such as, for example, without being limited thereto, a culture plate, preferably a low-volume culture plate, and, more preferably, having an adhesive plastic surface, in the presence of the cell culture medium of the invention, for 70-74 hours and, preferably, for 72 hours. Once this period of time has elapsed, inactivated endothelial cells are added to the culture of step (b). "Endothelial cells" are understood to mean the flat cells that coat the interior of blood vessels and, especially, the capillary vessels, forming a part of the wall, the nucleus whereof has a flat morphology and appears to be elliptical in the cuts visualised under the microscope. "Inactivated endothelial cells" are those endothelial cells that have lost their mitotic capacity. The endothelial cells of step (c) of the method of the invention are derived, preferably, from the Mil Seven 1 (MS 1) cell line, known to persons skilled in the art and available, for example, without being limited thereto, in the American Type Culture Collection (catalogue number CRL-2279), when the cells isolated in step (a) of the method of the invention are pancreatic beta cells. If the cells isolated in step (a) are hepatocytes, then the endothelial cells used will be derived, for example, without being limited thereto, from the liver itself. Inactivated endothelial cells may be obtained by means of, for example, without being limited thereto, the *in vitro* culture of endothelial cells with mitomycin C, preferably in a quantity ranging between 2 and 3 µg/ml, and, more preferably, in a quantity of 2.5 µg/ml, for preferably between 2 and 3 hours, and, more preferably, for 2 hours.

The inactivated endothelial cells are added in step (c) of the method of the invention to the culture of step (b), preferably at a density ranging between 50,000 and 70,000 cells/cm², and, more preferably, at a density of 60,000 cells/cm². Said cell density may be reached, in the case of cells derived from the MS 1 cell line, by culturing said cells, for example, without being limited thereto, in a DMEM 4.5 g/I glucose culture medium supplemented with between 3%-6% of foetal bovine serum, preferably with 5% of foetal bovine serum.

In step (d) of the method of the invention, the inactivated endothelial cells and the cells cultured in the culture medium of the invention in step (b) are co-cultured for 10-12 days, preferably for 11 days. As shown in the examples of the present invention, following this co-culture period, rapidly-proliferating cell colonies are obtained which present endocrine precursor markers, which demonstrates the viability of the method of the invention, as well as the fact that the cells obtained have not lost their phenotype.

The term "pancreas" refers to the glandular organ located in the digestive and endocrine systems of vertebrates. It is both an endocrine gland (it produces insulin, glucagon and somatostatin) and an exocrine gland (it secretes pancreatic juice, which contains digestive enzymes that go to the small intestine). It has a conical shape and, in the human species, its length ranges between 20 and 30 cm, it is approximately 4 cm wide and approximately 5 cm thick; its weight is approximately 30 g. A "pancreatic tissue" is any tissue that is a part of this organ.

"Islets of Langerhans" or "pancreatic islets" are understood to mean the cell accumuli that are preferably in charge of producing hormones such as insulin and glucagon, with a primarily endocrine function. They also secrete other hormones and peptides. They form small cell groups of about 1,500 to 3,000 cells, dispersed throughout the entire pancreas. In cuts stained with hematoxylin-eosin, they have the appearance of pale pink irregular islets extensively distributed amongst the darker-coloured exocrine acini.

"Pancreatic beta cells" are a type of pancreatic cells located in the islets of Langerhans which synthesise and secrete insulin.

The method of the invention makes it possible to obtain an *in vitro* expanded cell population of pancreatic beta cells from pancreatic beta cells isolated from an islet of Langerhans or from isolated islets of Langerhans obtained from an isolated or partially isolated pancreas.

In addition to the aforementioned steps of the method of the invention, said method may comprise other additional steps, for example, without being limited thereto, in relation to the isolation of cells of step (a).

Another aspect of the invention relates to a cell that is obtainable by means of the method of the invention, hereinafter "endodermal cell of the invention". In a preferred embodiment of this aspect of the invention, the endodermal cell of the invention is a pancreatic beta cell, hereinafter "pancreatic beta cell of the invention". In another preferred embodiment, the endodermal cell of the invention is a hepatocyte, hereinafter "hepatocyte of the invention".

Another aspect of the invention relates to a cell population that is obtainable by means of the method of the invention, hereinafter "endodermal cell population of the invention". In a preferred embodiment, the endodermal cell population of the invention is formed by pancreatic beta cells, hereinafter "pancreatic beta cell population of the invention". In another preferred embodiment, the endodermal cell population of the invention is formed by hepatocytes, hereinafter "hepatocyte population of the invention".

Another aspect of the invention relates to the use of the endodermal cell or cell population of the invention, preferably the pancreatic beta cell or the pancreatic beta cell population, the hepatocyte or the hepatocyte population of the invention, for the preparation of a drug. Another aspect of the invention relates to the use of the endodermal cell or cell population of the invention for the preparation of a drug designed for somatic cell therapy for injuries or diseases of endoderm-derived tissues.

Another aspect of the invention relates to the use of the pancreatic beta cell or cell population of the invention for the preparation of a drug designed for somatic cell therapy for injuries or diseases of the pancreas. In a preferred embodiment of this aspect of the invention, the pancreas disease is diabetes mellitus.

Another aspect of the invention relates to the use of the hepatocyte or the hepatocyte population of the invention for the preparation of a drug designed for somatic cell therapy for injuries or diseases of the liver.

The endodermal cell of the invention is a pancreatic beta cell when the cells isolated in step (a) of the method of the invention are pancreatic beta cells. The endodermal cell population of the invention is formed by pancreatic beta cells when the cells isolated in step (a) of the method of the invention are pancreatic beta cells. The endodermal cell of the invention is a hepatocyte when the cells isolated in step (a) of the method of the invention are hepatocytes. The endodermal cell population of the invention is formed by hepatocytes when the cells isolated in step (a) of the method of the invention are hepatocytes.

The term "drug", as used in this description, refers to any substance used for the prevention, diagnosis, relief, treatment or healing of diseases in humans and animals. Within the context of the present invention, it refers to a preparation that comprises, at least, the endodermal cell or cell population obtainable by means of the method of the invention, the pancreatic beta cell or cell population obtainable by means of the method of the invention, or the hepatocyte or the hepatocyte population obtainable by means of the method of the invention.

The term "treatment", as understood in the present invention, entails combatting the effects caused by injuries or diseases of endoderm-derived tissues, preferably injuries or diseases of the pancreas, more preferably, diabetes mellitus, in order to stabilise the condition of individuals or prevent subsequent damages.

"Somatic cell therapy" is understood to mean the use of living somatic cells, both autologous (from the patients themselves) and allogeneic (from another human being) or xenogeneic (from animals), the biological characteristics whereof may have been altered as a result of their manipulation, in order to obtain a therapeutic, diagnostic or preventive effect, by metabolic, pharmacological or immunological means. Drugs for somatic cell therapy include, for example, without being limited thereto: cells that are manipulated in order to qualitatively or quantitatively modify their immunological, metabolic or other type of functional properties; cells that are classified, selected and manipulated, and subsequently subjected to a manufacturing process in order to obtain the finished product; cells that are manipulated and combined with non-cellular components (for example, biological or inert matrices or sanitary products) that exert the action intended in principle from the finished product; derivatives of autologous cells expressed *ex vivo (in vitro)* under specific culture conditions; or genetically modified cells or cells subjected to another type of manipulation in order to express previously unexpressed homologous or non-homologous functional properties.

The cells, as well as the cell populations, that are obtainable by means of the method of the invention may be used, for example, without being limited thereto, for the prevention and/or regeneration of tissue injuries by transplanting them to the damaged organ, as well as for metabolic or genetic diseases. Said cells and cell populations may be used in cell therapy jointly with one or more compounds, drugs or cells useful for the prevention or treatment of injuries or diseases of endoderm-derived tissues, simultaneously or sequentially.

In the present invention, "injuries or diseases of endoderm-derived tissues" are understood to mean all those injuries or metabolic or genetic diseases that affect or are produced in endoderm-derived tissues.

One of the "injuries or diseases of the pancreas" for which the drugs of the present invention may be useful is, for example, without being limited thereto, diabetes mellitus.

The term "diabetes mellitus" refers to the group of metabolic disorders that affect different organs and tissues, last for life and are characterised by an increase in the blood levels of glucose: hyperglycaemia. It is caused by several disorders, including a low production of the insulin hormone, secreted by the pancreatic beta cells, the absence of or a significant decrease in the mass of beta cells, and/or an increase in insulin resistance. The main symptoms of diabetes mellitus are: excessive emission of urine (polyuria), abnormal increase in the need to eat (polyphagia), increased thirst (polydipsia) and loss of weight without an apparent reason. This term includes the types of diabetes mellitus selected from the list that comprises: diabetes mellitus type 1, diabetes mellitus type 2, gestational diabetes and other types of diabetes mellitus.

The "injuries or diseases of the liver" for which the drugs of the present invention may be useful are, for example, without being limited thereto, cirrhosis, hepatitis A, B or C, liver cancer, hepatocarcinoma, hepatocellular carcinoma or hepatoma, haemochromatosis, hepatic ischaemia or hepatopathy or hepatic disease.

Another aspect of the invention relates to a kit designed to obtain cells from isolated tissues of endodermal origin, hereinafter "kit of the invention", which comprises the cell culture medium of the invention. In a preferred embodiment, the kit of the invention further comprises inactivated endothelial cells. In a more preferred embodiment, the kit of the invention further comprises all the elements necessary to perform the digestion protocol of the invention.

Another aspect of the invention relates to the use of the kit of the invention to obtain cells from isolated tissues of endodermal origin.

Said kit may comprise, without any limitation whatsoever, culture media, sera, factors that favour the growth of cells under culture, such as amino acids, antibiotics, cytokines, etc., buffers, reagents, contamination prevention agents. It may further comprise primers, probes, polymerases, antibodies, reagents, etc., and, in general, the means necessary to perform cell characterisation. On the other hand, the kit may include the methods and means necessary to perform the extraction, purification, etc. of nucleic acids and/or proteins. It may also include all the media and containers necessary for the implementation and optimisation thereof. Preferably, the kit further comprises the instructions to implement the method of the invention. For illustrative purposes, without this limiting the scope of the invention, the kit will contain the necessary elements to obtain cells from isolated tissues of endodermal origin, using the technique described above.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and drawings are provided for illustrative purposes, and are not intended to limit the scope of the present invention.

### DESCRIPTION OF THE FIGURES

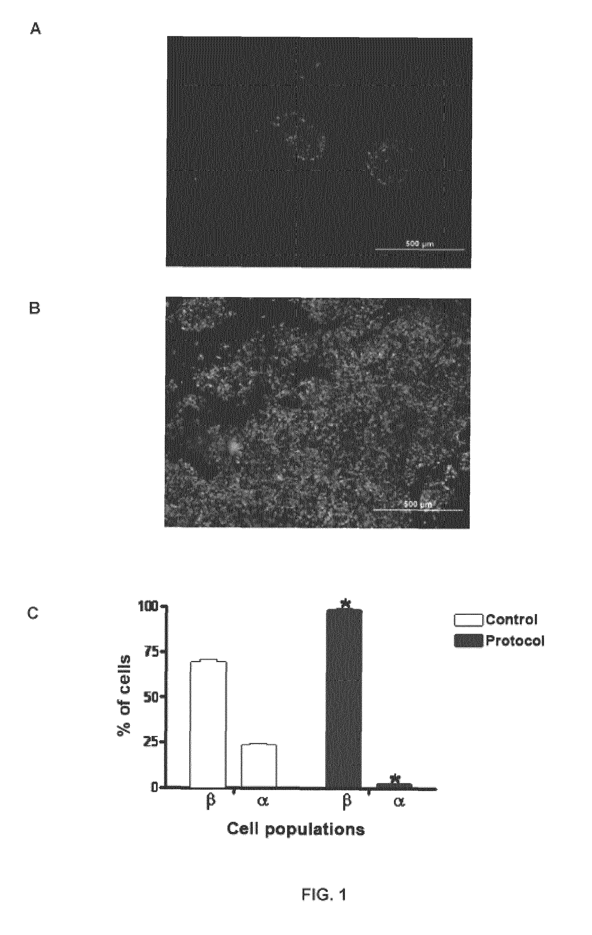

Fig. 1 shows the enrichment of pancreatic beta cells under culture in the presence of the proliferation-inducing medium. The cells that appear in the figure are insulin-positive. A: murine pancreatic islets cultured for 72 h in the control culture medium. B: enriched beta cell culture, cultured for 72 h in the proliferation-inducing culture medium. C: graph that shows the percentage of alpha and beta cells in the cultures for conditions A and B. The controls refer to condition A and the protocol refers to condition B. The results of a representative experiment of the 10 performed are shown. ^{*}p < 0.001 control vs protocol. Bar = 500 µm.

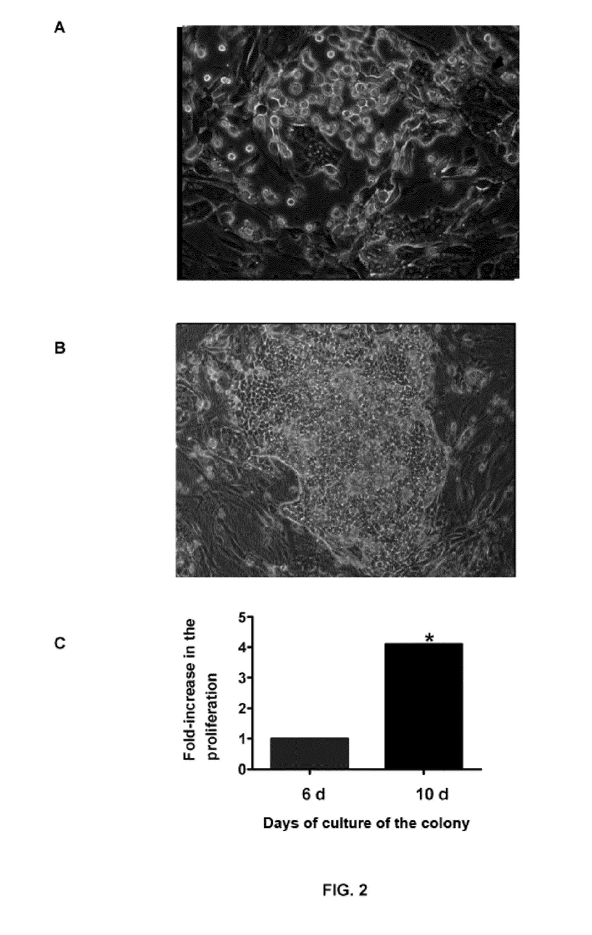

Fig. 2. shows the formation of rapidly-growing beta cell colonies at different days of culture, following co-culture with the inactivated endothelial cells. It is a phase-contrast image. A: formation of colonies after 6 days of culture. B: formation of colonies after 10 days of culture. C: graph that shows the increase in the proliferation in the colonies at 6 and 10 days of culture. The results of a representative experiment of the 10 performed are shown. **p* < 0.001 6 days vs 10 days. Bar = 50 µm.

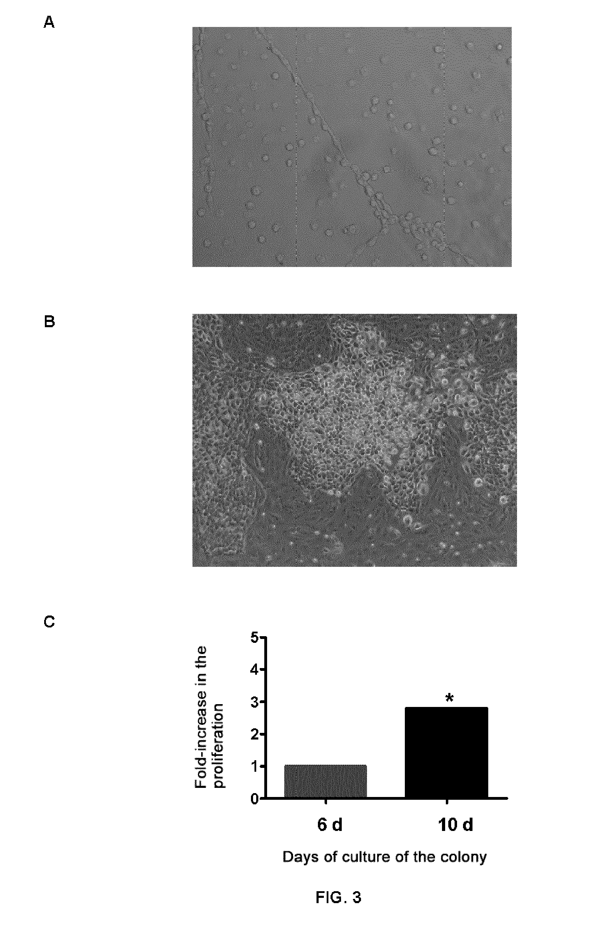

Fig. 3 shows the formation of rapidly-growing hepatocyte colonies at different days of culture, following co-culture with the inactivated endothelial cells. It is a phase-contrast image. A: formation of colonies after 6 days of culture. B: formation of colonies after 10 days of culture. C: graph that shows the increase in the proliferation in the colonies at 6 and 10 days of culture. The results of a representative experiment of the 10 performed are shown. **p* < 0.001 6 days vs 10 days. Bar = 50 µm.

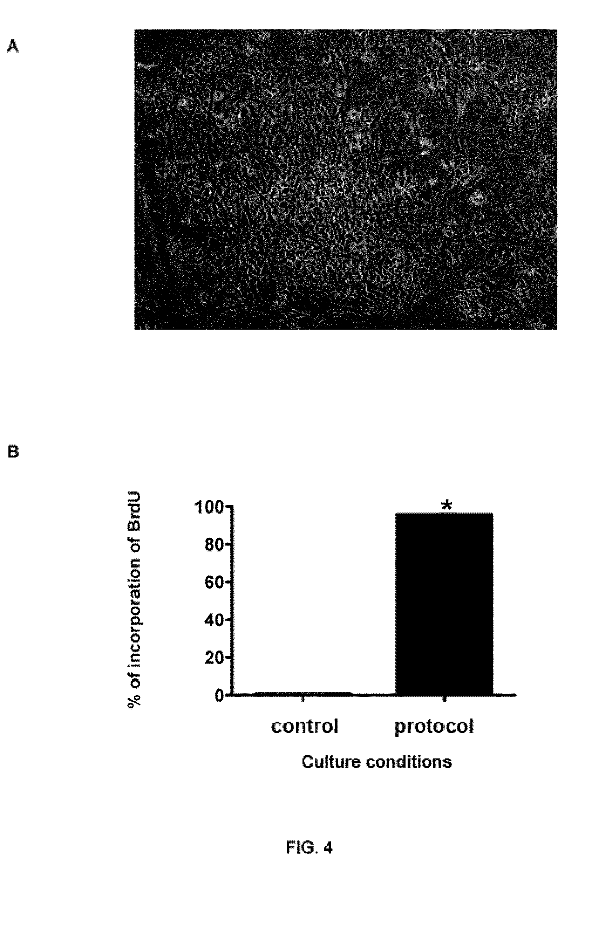

Fig. 4 shows the incorporation of BrdU in islets cultured in the control culture medium (control) and the proliferation-inducing medium (protocol), for 10 days. A: The cells that replicate incorporate BrdU. It is a colony after 10 days of culture. In this image, there is a superimposition of a fluorescence image with a phase-contrast image. B: Graph that shows the level of incorporation of BrdU in islets cultured in the control culture medium (control) and the proliferation-inducing medium (protocol), for 10 days. The results of a representative experiment of the 10 performed are shown. **p* < 0.001 control vs protocol. Bar = 50 µm.

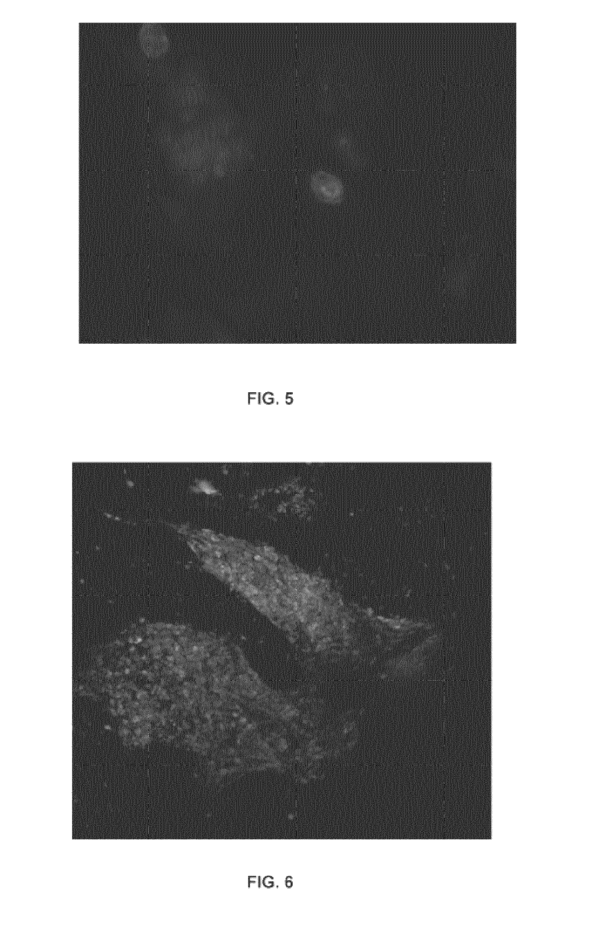

Fig. 5 shows an immunofluorescence image in islets cultured in the proliferation-inducing medium (protocol), for 14 days. The image shows insulin-positive cells and cytokeratin-19-positive cells. The nuclei are labelled with DAPI. It is a colony after 14 days of culture. The results of a representative experiment of the 3 performed are shown. Bar = 50 µm.
Fig. 6 shows an immunofluorescence image in islets cultured in the proliferation-inducing medium (protocol), for 14 days. The image shows neurogenin-3-positive cells and cytokeratin-19-positive cells. The nuclei are labelled with DAPI. It is a colony after 14 days of culture. The results of a representative experiment of the 3 performed are shown. Bar = 50 µm.

### EXAMPLES

Below we will illustrate the invention by means of assays performed by the inventors, which show the specificity and effectiveness of the method for obtaining cells from isolated tissues of endodermal origin developed in the invention. The following specific examples provided in this document serve to illustrate the nature of the present invention. These examples are included solely for illustrative purposes and should not be interpreted to be limitations to the invention claimed herein. Therefore, the examples described below illustrate the invention without limiting the field of application thereof.

### Example 1. In vitro proliferation of beta cells derived from pancreatic islets

Pancreatic islets from the OF1 mouse strain, males 8-12 weeks of age and weighing 35-40 g, were isolated by means of the islet isolation protocol developed by Lernmark (Lernmark A., 1974, Diabetologia, 10: 431-438), with one modification, by collagenase digestion. In the first place, an islet isolation solution was prepared. This solution was at 4°C. The composition of the buffer of the solution was (in mM): 115 NaCl, 5 KCl, 10 NAHCO₃, 1.1 MgCl₂, 1.2 NaH₂PO₄, 1 CaCl₂ and 0.5 glucose. 0.5% bovine albumin (Sigma) was added to this solution and the pH was adjusted to 7.3. 8 ml of this solution were collected and 6.3 mg of type V collagenase from Sigma (collagenase activity 533 units/mg) were added thereto. 3 ml of the cold collagenase solution were injected into the bile duct of the animal and, after the pancreas was swollen, it was extracted as quickly as possible, removing the fatty tissue and the connective tissue. Subsequently, the pancreas was placed in a tube with 7 ml of collagenase. Thereafter, the tube with the pancreas was introduced into an incubation bath at 37°C. The pancreas was vigorously stirred by hand, for 15 seconds, at minutes 5, 7 and 9. The incubation was stopped at minute 10 by the addition of 5 ml of isolation solution at 4°C, without collagenase. The resulting suspension was centrifuged 2 times at 200 g for 5 minutes, at room temperature. In each centrifugation, the supernatant was eliminated. Finally, using a pipette, the islets were manually picked under a dissecting magnifying glass and placed in a Petri dish, with cold isolation solution without collagenase. The islets that were not completely separated from the exocrine tissue or the vascular tissue were manually dissected with two insulin syringes. Following the isolation of the islets, these were washed in a Dulbecco phosphate buffer solution containing 1% of bovine albumin, 2.5 mM glucose and 1.1 mM MgCl₂. The islets in this wash solution were centrifuged once at 50 g, for 1 minute, at room temperature. Subsequently, the supernatant was eliminated and 3 ml of a non-enzymatic cell dissociation solution (Sigma, catalogue no. C5914), at 37°C, were added. Thereafter, the islets were incubated for 8 minutes in an incubation bath at 37°C. During the incubation time, the islets were mechanically dispersed by pipetting 10 consecutive times, with a 1-ml pipette, at minutes 2, 4 and 6. The pipetting must be soft and care must be taken not to produce bubbles. Durante the last minute of the digestion, 1 ml of a trypsin-EDTA (Gibco) 0.1 x solution was added and the mechanical dispersion was continued by means of pipetting. In order to stop the dissociation process of the pancreatic islets into cells, 5 ml of DMEM culture medium (Gibco), containing 4.5 g/I of glucose and supplemented with 10% of foetal bovine serum (Hyclone), were added. Subsequently, the cell suspension was centrifuged at 200 g for 5 minutes, at room temperature, the supernatant was eliminated and 1 ml of the aforementioned culture medium was added. Thereafter, the cell suspension was filtered through a 70-µm mesh (Becton Dickinson) using a 1-ml pipette at high velocity. Following the filtration, the cell suspension was centrifuged once again at 200 g for 5 minutes, at room temperature. Finally, the cells were seeded in low-volume culture plates, with an adhesive plastic surface (Greiner, catalogue no. 627170). The cells were seeded in 60 µl per well, at a density of 100,000 cells/cm².

The islets and the dissociated cells were cultured in the presence of different media, for 72 h. The islets were cultured in the control medium (Figure 1A), which was composed of DMEM 1 g/I glucose (Gibco), 5% foetal calf serum (Hyclone), 100 U/ml-100 µg/ml penicillin-streptomycin (Gibco). The dissociated cells were cultured in the proliferation-inducing medium (Figure 1B). This medium contained DMEM 1 g/I glucose (Gibco), 5% foetal calf serum (Hyclone), 100 U/ml-100 µg/ml penicillin-streptomycin (Gibco), 1 mM sodium pyruvate (Gibco), 1% non-essential amino acids (Gibco), 0.1 mM 2-beta-mercaptoethanol (Gibco), 0.1 µg/ml hydrocortisone (Sigma), 5 µg-5 µg-5 ng/ml insulin-transferrin-selenium (Roche), 1,000 U/ml mouse recombinant leukemia inhibitory factor (Chemicon), 10 ng/ml epidermal growth factor (Sigma) and 1 mM EGTA (Sigma). Following 72 h of culture, immunofluorescence was performed in order to detect the insulin-positive cells. As the primary antibody, monoclonal anti-insulin (Sigma, catalogue number 12018) was used, at a 1/400 dilution. As the secondary antibody, Alexa Fluor 488 (Invitrogen) was used, at a 1/400 dilution. The data showed that the culture method or protocol using the proliferation-inducing medium described herein makes it possible to obtain a culture that is practically pure in pancreatic beta cells (Figure 1C). Thus, it was possible to go from a culture with 69% ± 3% of beta cells under control conditions to a culture with 98% ± 2% of beta cells with the protocol of the invention (n = 10, *p* < 0.001). As regards alpha cells, it was possible to go from a culture with 23% ± 3% of alpha cells under control conditions to a culture with 2% ± 1 % of alpha cells with the protocol of the invention (n = 10, *p* < 0.001).

Subsequently, 60,000 cells/cm² of the Mil Seven 1 cell line (MS 1) were added to the beta-cell-rich cell culture after 3 days of culture in the proliferation-inducing medium. This is a capillary endothelial cell line that was acquired from the American Type Culture Collection (catalogue number CRL-2279). Previously, this cell line was inactivated by culturing it with 2.5 µg/ml of mitomycin C (Sigma) for 2 h. The culture medium for the line was DMEM 4.5 g/I glucose (Gibco) supplemented with 5% foetal bovine serum (Gibco).

Subsequently, the co-culture of the beta cells and the MS 1 capillary endothelial cells was maintained for 11 additional days in the presence of the proliferation-inducing medium. It was observed that, from day 6 of culture (Figure 2A), proliferating cell colonies began to form (n = 10). On day 10 of culture (Figure 2B), numerous rapidly-proliferating colonies with about 2,000 cells appeared (n = 10). The increase in the cell proliferation observed was 4.02 ± 0.3 times (n = 10, *p* < 0.001) (Figure 2C). Moreover, the three days under culture in the proliferation-inducing medium produced a significant proliferation of insulin-positive cells, as compared to the islets cultured in the control medium (Figure 1B, n = 10, *p* < 0.001).

### Example 2. In vitro proliferation of hepatocytes derived from hepatic tissue

In order to test this *in vitro* cell proliferation method in another tissue of endodermal origin, a very similar protocol was developed for hepatic tissue. To this end, hepatocytes were isolated by means of the traditional two-step collagenase digestion protocol, developed by Seglen (Seglen, P.O., Methods in Cell Biology, vol. XIII, David M. Prescott ed., Academic Press, 1976; Chapter 4, pp. 29-83), and cultured in the presence of endothelial cells from the liver itself. The cells were seeded in 60 µl per well, at a density of 100,000 cells/cm². The endothelial cells from the liver were at a density of 60,000 cells/cm². In this case, the culture medium was: DMEM 4.5 g/I glucose with glutamax (2 mM), sodium pyruvate (1 mM) and HEPES (10 mM) (Gibco), 10% foetal calf serum (Hyclone), 100 U/ml-100 µg/ml penicillin-streptomycin (Gibco), 0.1 mM 2-beta-mercaptoethanol (Gibco), 0.1 µg/ml hydrocortisone (Sigma), 5 µg-5 µg-5 ng/ml insulin-transferrin-selenium (Roche), 1,000 U/ml mouse recombinant leukemia inhibitory factor (Chemicon) and 1.5 mM EGTA (Sigma).

In the case of the culture of hepatocytes jointly with hepatic endothelial cells, it was observed that, from day 6 of culture (Figure 3A), very rapidly-proliferating cell groups began to form (n = 10). On day 10 of culture, very rapidly-proliferating colonies with about 1,500 cells appeared (n = 10) (Figure 3B). In this case, the increase observed in cell proliferation was 2.8 ± 0.2 times (n = 10, *p* < 0.001) (Figure 3C).

### Example 3. BrdU incorporation index in the cell colonies

After 10 days of culture in the proliferation-inducing medium, a BrdU incorporation study was performed in order to determine the degree of replication of the cells that were a part of the colonies. The incorporation of BrdU at 24 h was assessed by means of immunofluorescence. As the primary antibody, anti-BrdU (Abcam, catalogue number ab6326) was used, at a 1/400 dilution (Figure 4A). Figure 4B shows that, after 10 days of culture in the presence of the inducing medium, the incorporation of BrdU and, therefore, the replication in the proliferating colonies, was 96% ± 3% (n = 10). This increase in replication was highly significant, when compared to islets cultured in the control medium for 10 days, in which case the incorporation of BrdU was 0.9% ± 0.3% (n = 10) (Figure 4B, n = 10, *p* < 0.001).

### Example 4. Epithelial-mesenchymal transition of the cell colonies cultured in the presence of the inducing medium

After 14 days of culture in the inducing medium, a study of the degree of cellular disdifferentiation was performed. To this end, double immunocytochemistries were performed for insulin and cytokeratin 19 (Figure 5). Cytokeratin 19 is an epithelial tissue marker. For insulin, monoclonal anti-insulin (Sigma, catalogue number I2018) was used as the primary antibody, at a 1/200 dilution. As the secondary antibody, Alexa Fluor 594 (Invitrogen) was used, at a 1/300 dilution. For cytokeratin 19, polyclonal anti-cytokeratin 19 (Abcam, catalogue number ab15463) was used as the primary antibody, at a 1/200 dilution. As the secondary antibody, Alexa Fluor 488 (Invitrogen) was used, at a 1/300 dilution. In the image, it may be observed that the cells in the periphery of the clone were positive for insulin and cytokeratin 19 (n = 3). These data indicated that 3% ± 1% of the insulin-positive cells, i.e. of endothelial origin, were undergoing an epithelial-mesenchymal transition. These cells in the periphery of the clone were the ones that produced the proliferating cell colonies.

### Example 5. Labelling for endocrine precursors in the cells of the proliferating colonies

After 14 days of culture in the inducing medium, the presence of endocrine markers or endocrine precursor markers was studied. To this end, double immunocytochemistries were performed for neurogenin 3 and cytokeratin 19 (Figure 6). Neurogenin 3 is an endocrine cell precursor marker. For neurogenin 3, monoclonal anti-neurogenin 3 (Beta Cell Biology Consortium, catalogue number AB2013) was used as the primary antibody, at a 1/50 dilution. As the secondary antibody, Alexa Fluor 488 (Invitrogen) was used, at a 1/300 dilution. For cytokeratin 19, polyclonal anti-cytokeratin 19 (Abcam, catalogue number ab15463) was used as the primary antibody, at a 1/200 dilution. As the secondary antibody, Alexa Fluor 594 (Invitrogen) was used, at a 1/300 dilution. In the image, it may be observed that 95% ± 4% of the cells in the colony simultaneously expressed neurogenin 3 and cytokeratin 19 (n = 3). This co-location, in all the cells in the colony, indicated that, although the rapidly-replicating cells in the colony underwent a disdifferentiation process, they had endocrine precursor cell properties. I.e. a population of rapidly-proliferating cells with endocrine precursor characteristics was being obtained.

## Claims

1. DMEM cell culture medium supplemented with:
a. between 4% and 10% of "foetal calf serum",
b. between 90 and 110 U/ml of penicillin, and between 80 and 120 µg/ml of streptomycin,
c. between 0.5 and 2 mM of sodium pyruvate,
d. between 0.05 and 0.2 mM of 2-beta-mercaptoethanol,
e. between 0.05 and 0.2 µg/ml of hydrocortisone,
f. between 4 µg-4 ng/ml and 6 µg-ng/ml of insulin-transferrin-selenium,
g. between 900 and 1,100 U/ml of leukaemia inhibitory factor,
h. between 1 and 2 mM of EGTA,
i. between 0.5% and 2% of non-essential amino acids, and
j. between 9 and 11 ng/ml of epidermal growth factor.

2. Cell culture medium of claim 1 wherein the DMEM medium comprises 1 g/I glucose.

3. Method for obtaining cells from isolated tissues of endodermal origin comprising:
a. isolating the cells of a biological sample isolated from a tissue of endodermal origin of a mammal using a collagenase digestion protocol,
b. culturing the cells isolated in step (a) in the presence of the culture medium of any of the claims 1 or 2 for 70 - 74 hours,
c. adding inactivated endothelial cells to the culture of step (b), and
d. co-culturing the culture of step (b) and the inactivated endothelial cells added in step (c) for 10 - 12 days.

4. Method of claim 3 wherein the density of the cultured cells in step (b) is between 90,000 and 110,000 cells/cm² at the start of culture.

5. Method of any of the claims 3 or 4 wherein the density of inactivated endothelial cells of step (c) is between 50,000 and 70,000 cells/m² at the time of addition to the culture of step (b).

6. Method of any of the claims 3 to 5 wherein the tissue of endodermal origin of step (a) is pancreatic tissue.

7. Method of any of the claims 3 to 6 wherein the isolated biological sample of step (a) is an islet of Langerhans.

8. Method of any of the claims 3 to 7 wherein the cells isolated in step (a) are pancreatic beta cells.

9. Method of any of the claims 3 to 8 wherein the inactivated endothelial cells of step (c) are derived from the Mil Seven 1 cell line.

10. Method of any of the claims 6 to 9 wherein the collagenase digestion protocol of step (a) comprises:
a. preparing a solution that comprises 115 mM NaCl, 5 mM KCl, 10 mM NAHCO₃, 1.1 mM MgCl₂, 1.2 mM NaH₂PO₄, 1 mM CaCl₂, between 0.5 and 1 mM of glucose, and between 0.5% and 3% of bovine albumin, at 4 °C and pH 7.3,
b. adding between 5 and 8 mg of type V collagenase to between 6 and 10 ml of the solution of step (a),
c. placing the pancreas, previously extracted from a mammal that had been injected with between 2.5 and 5 ml of the solution of step (b), in a container with between 5 and 9 ml of collagenase,
d. incubating the container of step (c) at 37 °C and stirring it for 15 seconds at minutes 5, 7 and 9,
e. stopping the incubation of step (d) at minute 10 by the addition of between 3 and 6 ml of the solution of step (a),
f. centrifuging the solution obtained in step (e) twice at 200 g for between 3 and 5 minutes at room temperature and discarding the supernatant after each centrifugation,
g. isolating the pancreatic islets present in the solution obtained in step (f), by manual picking with a pipette under a stereoscopic magnifying lens, and placing them in a container with the solution of step (a),
h. adding a phosphate buffer solution that comprises between 0.5% and 3% of bovine albumin, between 1 and 3 mM glucose, and 1.1 mM MgCl₂ to the container of step (g),
i. centrifuging the solution of step (h) at 50 g for between 1 and 3 minutes at room temperature, and discarding the supernatant,
j. adding between 2 and 4 ml of a non-enzymatic cell dissociation solution to the solution obtained in step (i) at 37 °C,
k. incubating the solution of step (j) at 37 °C for 8 minutes and mechanically dispersing the pancreatic islets in the solution by means of 10 pipettings at minutes 2, 4 and 6 of said incubation,
l. adding between 0.5 and 1.5 ml of a trypsin-EDTA solution to the solution of step (k) during the last minute of the incubation,
m. adding between 3 and 6 ml of DMEM culture medium to the solution obtained in step (I),
n. centrifuging the solution of step (m) at 200 g for between 3 and 5 minutes at room temperature, and eliminating the supernatant,
o. adding between 1 and 2 ml of the culture medium of step (m) to the solution obtained in step (n),
p. filtering the solution obtained in step (o) through a 70-µm mesh,
q. centrifuging the filtrate obtained in step (p) at 200 g for between 3 and 5 minutes at room temperature, and
r. seeding the cells obtained following the centrifugation of step (q).

11. Cell obtainable by means of a method of any of the claims 3 to 10.

12. Cell of claim 11 that is a pancreatic beta cell.

13. Cell population obtainable by means of a method of any of the claims 3 to 10.

14. Cell population of claim 13 wherein the cells are pancreatic beta cells.

15. Use of the cell of any of the claims 11 or 12 or of the cell population of any of the claims 13 or 14 for the preparation of a medicinal drug.

16. Use of the cell of claim 11 or of the cell population of claim 13 for the preparation of a medicinal drug for somatic cell therapy of injuries or diseases of endoderm-derived tissues.

17. Use of the cell of claim 12 or of the cell population of claim 14 for the preparation of a medicinal drug for somatic cell therapy of injuries or diseases of the pancreas.

18. Use of the cell or of the cell population of claim 17 wherein the disease of the pancreas is diabetes mellitus.

19. Kit for obtaining cells from isolated tissues of endodermal origin comprising a cell culture medium of any of the claims 1 or 2.

20. Kit of claim 19 that additionally comprises inactivated endothelial cells.

21. Kit of any of the claims 19 or 20 that additionally comprises all the elements necessary to carry out the collagenase digestion protocol of claim 10.

22. Use of the kit of any of the claims 19 to 21 for obtaining cells from isolated tissues of endodermal origin.
